Europäisches Patentamt

European Patent Office

Office européen des brevets

(11) Veröffentlichungsnummer: **0 263 934**
**A1**

## (12) EUROPÄISCHE PATENTANMELDUNG

(21) Anmeldenummer: 87111312.2

(51) Int. Cl.⁴: **B01D 15/08**

(22) Anmeldetag: **05.08.87**

(30) Priorität: **09.08.86 DE 3627063**

(43) Veröffentlichungstag der Anmeldung:
**20.04.88 Patentblatt 88/16**

(84) Benannte Vertragsstaaten:
**AT BE CH DE FR GB IT LI LU NL SE**

(71) Anmelder: **DIAGEN Institut für
molekularbiologische Diagnostik GmbH
Niederheider Strasse 3
D-4000 Düsseldorf 13(DE)**

(72) Erfinder: **Colpan, Metin, Dr.
Karschhauser Strasse 18
D-4006 Erkrath 2(DE)**

(74) Vertreter: **Werner, Hans-Karsten, Dr. et al
Deichmannhaus am Hauptbahnhof
D-5000 Köln 1(DE)**

(54) Methode zur Trennung und Reinigung von Biopolymeren durch Affinitätschromatographie.

(57) The method for the separation and purification of biopolymers by means of affinity chromatography using porous silica gel having affinity ligands covalently bonded to the surface thereof is improved by using silica gels which have defined narrow pore size ranges five to twenty times the size of the material to be purified.

EP 0 263 934 A1

# METHOD FOR SEPARATION AND PURIFICATION OF BIOPOLYMERS BY AFFINITY CHROMATOGRAPHY

The present invention relates to a method for the separation and purification of biopolymers by affinity chromatography using porous silica gel having affinity ligands covalently bonded to the surface thereof.

For affinity chromatography there are employed in the first place porous carriers such as dextrans, agaroses, acrylamide etc. which have pore size distributions of between 20 and 100 nm (200 and 1,000 Å). To these materials there are bound, for examples, covalent antibodies. By using these materials it is possible to purify proteins such as insulin, interferons etc. on a commercial scale.

In the U.S. Patent 4,415,665 there is described a specific method for covalently bonding biologically active organic substances to polymer carriers which include i.a., agarose, cellulose and silica gel. In Examples 4 and 5 there was used a porous silica gel of undefined pore size and undefined particle size. Covalently bonded was $N^6$-(6-amino-hexyl)-adenosine-5'-monophosphate. However, this method is not suitable for affinity chromatography of recombinant proteins like t-PA, urokinase, human growth hormone, blood factor VIII and monoclonal antibodies.

It is the object of the present invention to improve the method for the separation and purification of biopolymers by affinity chromatography using porous silica gel or other porous carriers (e.g. porous glass, kieselgur, alumina, titanium oxide and mixtures thereof) having affinity ligands covalently bonded to the surface thereof so that a rapid, reliable and highly specific separation and purification of biopolymers is possible.

The object is, on the one hand, an optimum utilization of the usually very expensive affinity ligand, such as, for example an antibody, and on the other hand, to keep the amount of carrier material as low as possible in order to avoid unnecessary losses and dilutions. Intensive investigations of various porous carriers, various affinity ligands and various biopolymers to be purified by affinity chromatography led to the surprising result that the object mentioned above can be attained in an optimum manner by using silica gels having defined narrow pore size ranges which have five to twenty times the size of the material to be purified.

If silica gels having undefined and wide pore size ranges are used, no satisfactory results can be obtained so that none of the objectives as mentioned above will be reached. If silica gels are used having defined narrow pore size ranges which, although they are larger than the material to be purified, are smaller than five times the size of the material to be purified, then the expensive and valuable affinity ligands are only incompletely utilized. Also, with smaller pore size ranges the amount of exuded affinity ligands and/or contaminating fragments thereof increases. If silica gels with pore size ranges larger than twenty times the size of the material to be purified are used, the surface of the material is decreased to such an extent that unnecessarily high amounts of silica gel and high volume of eluant liquid must be employed for subsequently eluting the biopolymer initially bonded. This is very undesirable, especially in the case of biopolymers to be used for therapeutical purposes.

Within the pore size range according to the invention of from five to twenty times the size of the material to be purified, the lower part of this range is preferred for use with virtually spherically biopolymers, while the upper part of this range is preferred for use in connection with long and bulky biopolymers.

Furthermore, the lower part of the pore size range is preferred where relatively small affinity ligands are bonded to the silica gel surface through relatively short spacers, while the upper part of this range is preferred where larger and bulkier affinity ligands and/or long-chain spacers are employed.

The affinity ligands covalently bonded to the surface include especially antibodies, but also other more or less voluminous substances having a specific affinity to the biopolymer to be purified. One typical example thereof is protein A of Staphylococcus aureus or the recombinant protein A which has a specific affinity to immunoglobulines (IgG).

The method according to the invention is of particular importance for the separation and purification of proteins, glycoproteins and/or nucleic acids, of viruses or vaccines, of cell organelles, prokaryotic or eukaryotic cells, micelles or vesicles, of chylomicrons, VLDL (very low density), LDL (low density) and HDL (high density) lipoproteins as well as protein complexes.

The invention is thus especially applicable to biopolymers of from at least 5 to 10 nm (50 to 100 Å) in size up to 500 nm (5,000 Å) and more. Thus, according to the invention there are employed porous silica gels having defined narrow pores size ranges of at least 30 nm (300 Å), however, preferably of between 50 and 1,000 nm (500 and 10,000 Å). In principle it would also be possible to employ silica gels having even larger pore sizes, however, it has so far been technically difficult to produce silica gels having narrow pores size ranges of this order of magnitude.

Some typical fields of application for the method according to the present invention are shown hereinbelow:

Viruses und Vaccines for Producing Materials for Vaccination

-Living vaccines (smallpox viruses, φ about 250 nm (2,500 Å))
-Attenuated virus strains (foot-and-mouth disease viruses, φ about 250 nm (2,500 Å))
-Recombinant vaccines prepared by gene technological expression methods from higher cell cultures or bacteria (e.g. hepatitis B virus coat protein complexes, φ about 50 nm (500 Å))
-Vaccines based on vesicles and micelles (φ about 10 to 50 nm (100 - 500 Å))

Organelles

- Mitochondria (φ about 500 nm (5,000 Å))
-Microbodies

Pro-and eucaryotic cells

-e.g. removal of mycoplasm (φ about 100 nm (1,000 Å)) and tumor cells (φ about 10,000 nm (100,000 Å)) from media or blood

Proteins

-Small proteins: Lymphokines such as, e.g., Interferon and TNF (MW 10,000 - 20,000)
-Medium size proteins: tissue-specific plasminogenactivators (MW about 65,000), IgG (MW 150,000, φ about 15 nm (150 Å)), urokinase (φ about 9 nm (90 Å))
-Large proteins: IgM (MW about 900,000, φ about 90 nm (900 Å)), blood factors such as, e.g., Factor VIII (MW > 1 million, φ > 50 nm (500 Å))

Protein complexes

-Removal of immunocomplexes such as Rheuma factors (MW 500,000, φ about > 50 nm (500 Å)) by washing blood
-Enzyme complexes such as fatty acid synthetase (MW about 2.3 millions)
-Filamentous complexes such as microtubulins, actin complexes
-Ribosomes

Micelles and Vesicles

-Chylomicrons (φ about 100 to 1,000 nm (1,000 - 10,000 Å))
-VLDL (very low density lipoproteins), φ about 30 to 50 nm (300 - 500 Å)
-LDL (φ about 20 to 25 nm (200 - 250 Å))
-HDL (φ > 10 nm (100 Å)

Covalent bonding of the affinity ligands to the surface of the pores of the silica gel is effected in a per se known manner, for example by reaction of a silane which can be activated at the organic moiety by subsequent reactions to react with the affinity ligand.

A particular mild and, thus, preferred method of activation consists of the preparation of alkyl or aryl sulfonic acid esters which react in a particularly mild manner with amino groups, hydroxyl groups and sulfhydryl groups of the affinity ligands to bind them through covalent bonds to the silica gel under mild conditions. However, basically all other known coupling reagents are also suitable (e.g. activation of amine groups with glutaraldehyde, hydroxyl groups with carbonyldiimidazcle, chloroformates, bisepoxyranes and diol groups with periodate) , as long as they do not result in providing the affinity ligands with an electrical charge or otherwise cause a denaturation to occur so as to affect the affinity. In principle, shorter as well as longer spacers may be inserted between the silica gel and the affinity ligands. However, spacers having only from 2 to 12 carbon atoms are particularly preferred. Use of shorter spacers may lead to unnecessary sterical hindrance of the reaction between the affinity ligand and the biopolymer to be purified. Longer spacers result in an unnecessary waste of the free space available within the pores without increasing the efficiency of the affinity ligands.

In the following Reference Examples there is described the preparation of some silica gels which have been activated at the surface thereof so that they are capable of covalently bonding various affinity ligands under mild conditions. In the subsequent Examples there is described how specific affinity ligands are covalently bonded to said surface and how the resulting porous silica gels may be used for an efficient affinity chromatography of the respective biopolymers.

Reference Example 1

Synthesis of Diol-Silica gel

In a 500 ml three-neck flask, 50 g of porous silica gel having a pore size of 100 nm (1,000 Å) and a particle size of about 60 microns are dried at a pressure of < 1 mbar at a temperature of 150 °C for 12 hours. Then the material is allowed to cool, the flask is aerated and 50 ml of distilled γ-glycidyloxypropyl-trimethoxysilane in 200 ml of absolute toluene and 1 ml tributylamine are added. The reaction is carried out at 111 °C, the reflux temperature of toluene, for 12 hours. Upon completion of the reaction the product, epoxy-silica gel, is filtered off with suction and is washed two times with 250 ml of toluene, three times with 250 ml of acetone and two times with 0.005 M HCl and then filtered off with suction. The epoxy-silica gel is suspended in 250 ml of 0.005 M HCl and is maintained at 90 °C for 2 hours to form the diol group.

The product, diol-silica gel, is collected by filtration with suction, washed five times with H₂O and two times with acetone and dried at 50 °C.

Reference Example 2

Activation of Diol-Silica gel with Tosyl Chloride

In a 500 ml three-neck flask, 50 g of dry diol-silica gel synthesized according to Reference Example 1 and having a pore size of 100 nm (1,000 Å) are dried at a pressure of < 1 mbar at 70 °C for 2 hours to remove any moisture if present. After drying, the flask is aerated, and 250 ml of absolute acetone and 1 ml of absolute pyridine are added. The suspension is degassed under vacuum and connected to a stirrer. To this suspension 10 mmoles of tosyl chloride, dissolved in 50 ml of absolute acetone are slowly added dropwise with constant stirring. After the reaction at room temperature of from 20 °C to 24 °C for 30 minutes, the tosyl-activated silicagel is filtered off by suction and washed with acetone, mixtures of acetone : 2 mM HCl having the compositions 75:25, 50:50 and 25:75 and finally with 1 mM HCl, filtered off by suction and freeze-dried.

Reference Example 3

Activation of Diol-Silica gel with Trichloromethanesulfonic Acid Chloride

In a 500 ml three-neck flask, 50 g of dry diol-silica gel synthesized according to Reference Example 1 and having a pore size of 250 nm (2,500 Å) are dried at a pressure of < 1 mbar at 70 °C for 2 hours to remove any moisture if present. After drying, the flask is aerated, and 250 ml of absolute acetone and 1 ml of absolute pyridine are added, and the equipment is connected to a stirrer. The suspension is degassed under vacuum and cooled in an ice bath to 0 °C. To this suspension 10 mmoles of trichloromethanesulfonic acid chloride, dissolved in 50 ml of absolute acetone are slowly added dropwise with permanent stirring. After the reaction at 0 °C for 30 minutes, the trichloromethane sulfonic acid chloride-activated silica gel is filtered off by suction and washed with acetone, mixtures of acetone : 2 mM HCl having the compositions 75:25, 50:50 and 25:75 and finally with 1 mM HCl, and freeze-dried.

Reference Example 4

Activation of Diol-Silica gel with 2,4,6-Trinitrobenzenesulfonic Acid Chloride

In a 500 ml three-neck flask, 50 g of dry diol-silica gel synthesized according to Reference Example 1 and having a pore size of 500 nm (5,000 Å) are dried at a pressure of < 1 mbar at 70 °C for 2 hours to remove any moisture if present. After drying, the flask is aerated, and 250 ml of absolute acetone and 1 ml of absolute pyridine are added, and the equipment is connected to a stirrer. The suspension is degassed under vacuum and cooled in an ice bath to 0 °C. To this suspension 10 mmoles of 2,4,6-trinitrobenzensulfonic acid chloride, dissolved in 50 ml of absolute acetone are slowly added dropwise with permanent stirring. After the reaction 0 °C for 30 minutes, the trinitrobenzene sulfonic acid chloride-activated silica gel is filtered of by suction and washed with acetone, mixtures of acetone : 2 mM HCl having the compositions 75:25, 50:50 and 25:75 and finally with 1 mM HCl, and freeze-dried.

Reference Example 5

Activation of Diol-Silica gel with 2,2,2-Trifluoroethanesulfonic Acid Chloride (Tresyl Chloride)

In a 500 ml three-neck flask, 50 g of dry diol-silica gel synthesized according to Reference Example 1 and having a pore size of 1,000 nm (10,000 Å) are dried at a pressure of < 1 mbar at 70 °C for 2 hours to remove any moisture if present. After drying, the flask is aerated, 250 ml of absolute acetone and 1 ml of absolute pyridine are added and the equipment is connected to a stirrer. The suspension is degassed under vacuum and cooled in an ice bath to 0 °C. To this suspension 10 mmoles of tresyl chloride are slowly added dropwise with permanent stirring. After the reaction at 0 °C for 30 minutes, the tresyl chloride-activated silica gel is filtered off by suction and washed with acetone, mixtures of acetone : 2 mM HCl having the compositions 75:25, 50:50 and 25:75 and finally with 1 mM HCl, and freeze-dried.

Reference Example 6

Activation of Diol-Silica gel with sodium periodate

In a 500 ml three-neck flask, 20 g Diol-Silica gel synthesized according to Reference Example 1 and having a pore size of 250 nm (2,500 Å) and particle size between 75 microns and 125 microns is suspended in 250 ml 0.1 M sodiumacetate, pH 4 and degassed under vacuum. 10 g sodium periodate dissolved in 50 ml water is added dropwise with constant stirring the suspension. The reaction is continued for 2 hours at room temperature. The Aldehyde-Silica gel is filtered by suction and washed several times with water, methanol and ether and dried under reduced pressure.

Example 1

Purification of Human Immunoglobulins (IgG) by Protein A Affinity Chromatography

A: 10 g of sulfonylchlorid-activated silica gel having a pore-size of 100 nm (1,000 Å) are suspended in 50 ml of 0.1 M Na-phosphate buffer, pH 8.0, and degassed under vacuum. To this suspension there are added 300 mg of Protein A (from Staphylococcus aureus or recombinant Protein A) dissolved in 15 ml of 0.1 M Na-Phosphate buffer, pH 8.0. The suspension is shaken at 4 °C for 6 hours to ensure a high degree of immobilization of protein A. After the reaction, the product protein A-silica gel is collected by filtration, suspended in 100 ml of 0.1 M mercaptoethanol, 0.1 M Na-phosphate buffer, pH 8.0, and shaken for 2 hours to block any unreacted sulfonyl groups. After the blocking reaction the product is washed five times with 0.1 M Na-phosphate buffer, pH 7.0.

B: 10 g of Protein A-Silica gel-1.000 (1.000 Å) are packed into a 1,27 cm × 15 cm chromatography column with inlet and outlet adapters and are washed two times with 3 column volumes of 0.1 M Na-phosphate buffer, pH 7.0, and 2 column volumes of 0.05 M Na-citrate buffer, pH 3.0, and equilibrated with 0.1 M Na-phosphate buffer, pH 7.0. The IgG sample consisting of 1,000 mg of crude human-IgG was dissolved in 100 ml 0.1 M Na-phosphate buffer, pH 7.0, and adsorbed onto the column with a flow rate of 10 ml/min. Contaminations of other proteins and unspecifically bound IgG are eluted with 2 column volumes of 0.1 M Na-phosphate buffer, pH 7.0, and the specifically bound human-IgG is eluted with 0.05 M of citrate buffer, pH 3.0. and at a flow rate of 1 ml/min.

The binding capacity of protein A-silica gel (pore size 1,000 Å) was determined to be 25 mg IgG/ml column volume.

Example 2

Purification of Human-Immunglobulins (IgG) with Anti-Human-IgG Antibodies by Affinity Chromatography

A: 200 mg of anti-human-IgG antibody are immobilized on 10 g of sulfonyl-activated silica gel having a pore-size of 250 nm (2.500 Å) in the same manner as in Example 1A.

B: In the same manner as in Example 1B the anti-human-IgG silica gel-2500 is packed into a chromatography column and equilibrated. After binding crude human-IgG, the nonspecifically bound proteins are eluted with 0.1 M Na-phosphate buffer, pH 7.0, and the specifically bound human-IgG is eluted with 0.05 M of citrate buffer, pH 3.0. at a flow rate of 1 ml/min.

The binding capacity of anti-human-IgG silica gel (pore size 250 nm (2,500 Å)) was determined to be 10 mg human IgG/ml column volume.

Example 3

Purification of Human IgM with Mouse Anti-Human-IgM Antibody Affinity Chromatography

A: 10 mg of anti-human-IgM antibody are immobilized on 10 g of sulfonyl-activated silica gel having a pore-size of 1,000 nm (10.000 Å) in the same manner as in Example 1A.

B: In the same manner as in Example 1B the anti-human-IgM silica gel-10000 is packed into a chromatography column and equilibrated. After binding crude human-IgM, the nonspecifically bound proteins are eluted with 0.1 M Na-phosphate buffer, pH 7.0, and the specifically bound human-IgM is eluted with 0.05 M of citrate buffer, pH 3.0. at a flow rate of 1 ml/min.

The binding capacity of anti-human-IgM silica gel (pore size 1,000 nm (10,000 Å)) was determined to be 0.75 mg human IgM/ml column volume.

Ansprüche

1. Method for the separation and purification of biopolymers by means of affinity chromatography using porous silica gel, porous glass, kieselgur, aluminumoxide, titaniumoxide, hydroxylapatite, zirconiumoxide or mixtures thereof having affinity ligands covalently bonded to the surface thereof, characterized in that silica gels are used which have defined narrow pore size ranges which have five to twenty times the size of the material to be purified.

2. The method according to claim 1, characterized in that silica gels are used which have defined narrow pore size ranges within a range of from 50 to 1,000 nm.

3. The method according to claim 1, characterized in that antibodies are used as affinity ligands.

4. The method according to claim 1, characterized in that the material to be purified is a protein, glycoprotein and/or nucleic acid.

5. The method according to claim 1, characterized in that the material to be purified is a virus or vaccine.

6. The method according to claim 1, characterized in that the material to be purified is a cell organelle.

7. The method according to claim 1, characterized in that the material to be purified is a prokaryotic or eukaryotic cells.

8. The method according to claim 1, characterized in that the material to be purified is a micelle, vesicle, chylomicron, VLDL, LDL and/or HDL lipoprotein.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | WO-A-8 303 363 (D. RIESNER et al.)<br>* claims 1, 15-17; page 2, lines 12-19; page 3, lines 8-16 * | 1,2,4,5 | B 01 D 15/08 |
| A | FR-A-2 403 556 (INSTITUT MERIEUX)<br>* claims 1, 2; page 2, lines 18-22; example 2 * | 1-3 | |
| A,D | US-A-4 415 665 (K.H. MOSBACH et al.)<br>* complete document * | | |
| A | FR-A-2 403 098 (INSTITUT MERIEUX)<br>* claims 1, 2, 12 * | | |

TECHNICAL FIELDS SEARCHED (Int. Cl.4)

B 01 D 15/00
B 01 J 20/00

The present search report has been drawn up for all claims

| Place of search | Date of completion of the search | Examiner |
|---|---|---|
| BERLIN | 19-12-1987 | BERTRAM H E H |

EPO FORM 1503 03.82 (P0401)